(19) Europäisches Patentamt
European Patent Office
Office européen des brevets

(11) Veröffentlichungsnummer: **0 046 931**
**A2**

(12) **EUROPÄISCHE PATENTANMELDUNG**

(21) Anmeldenummer: **81106468.2**

(22) Anmeldetag: **20.08.81**

(51) Int. Cl.³: **C 07 C 103/375**
C 07 D 307/68, A 01 N 37/46
A 01 N 43/08

(30) Priorität: **03.09.80 DE 3033160**

(43) Veröffentlichungstag der Anmeldung:
**10.03.82 Patentblatt 82/10**

(84) Benannte Vertragsstaaten:
**AT BE CH DE FR GB IT LI NL SE**

(71) Anmelder: **BAYER AG**
**Zentralbereich Patente, Marken und Lizenzen**
**D-5090 Leverkusen 1, Bayerwerk(DE)**

(72) Erfinder: **Stetter, Jörg, Dr.**
**Gellertweg 4**
**D-5600 Wuppertal 1(DE)**

(72) Erfinder: **Führer, Wolfgang, Dr.**
**Wehrstrasse 28**
**D-5202 Hennef 1(DE)**

(72) Erfinder: **Brandes, Wilhelm, Dr.**
**Eichendorffstrasse 5**
**D-5653 Leichlingen 1(DE)**

(54) **N-Oxiamide, Verfahren zu ihrer Herstellung sowie ihre Verwendung als Fungizide.**

(57) Die Erfindung betrifft neue N-Oxamide der Formel (I)

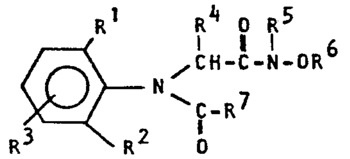

in der die Reste $R^1$ bis $R^7$ die in der Beschreibung angegebene Bedeutung haben,
ein Verfahren zu ihrer Herstellung und die Verwendung der erfindungsgemäßen Verbindungen als Fungizide.



Croydon Printing Company Ltd.

BAYER AKTIENGESELLSCHAFT 5090 Leverkusen-Bayerwerk
Zentralbereich
Patente, Marken und Lizenzen Slr/ABc
Ib

# N-Oxiamide, Verfahren zu ihrer Herstellung sowie ihre Verwendung als Fungizide

Die vorliegende Erfindung betrifft neue N-Oxiamide, mehrere Verfahren zu ihrer Herstellung und ihre Verwendung als Fungizide.

Es ist bereits bekannt geworden, daß Halogenacetanilide, wie beispielsweise N-Chloracetyl-N-(2,6-dimethylphenyl)-alanin- bzw. -glycin-alkylester mit gutem Erfolg zur Bekämpfung von pilzlichen Pflanzenkrankheiten eingesetzt werden können (vergleiche DE-OS 2 350 944 bzw. US-Patentschrift 3 780 090). Deren Wirkung ist jedoch, insbesondere bei niedrigen Aufwandmengen und -konzentrationen, insbesondere auch bei der Bekämpfung von Phytophthora-Arten, nicht immer ganz befriedigend.

Es wurden neue N-Oxiamide der allgemeinen Formel I gefunden:

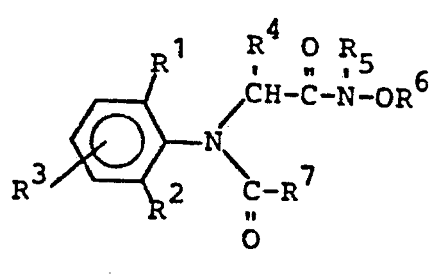

(I)

in welcher

$R^1$ für Wasserstoff, Alkyl oder Halogen steht,

$R^2$ für Wasserstoff oder Alkyl steht,

$R^3$ für Wasserstoff oder Alkyl steht,

$R^4$ für Wasserstoff oder Alkyl steht,

$R^5$ für Wasserstoff, gegebenenfalls durch Halogen substituiertes Alkyl, Alkenyl, Alkinyl steht,

$R^6$ für Wasserstoff, gegebenenfalls durch Halogen substituiertes Alkyl, Alkenyl, Alkinyl steht, wobei $R^5$ und $R^6$ auch eine Alkylenbrücke bilden können,

$R^7$ für Furyl, Tetrahydrofuryl, Thienyl, Tetrahydrothienyl, gegebenenfalls durch Halogen, Cyano oder Thiocyano substituiertes Alkyl, Alkenyl und Alkinyl; sowie die Gruppierungen $-CH_2-Az$, $-CH_2-OR^8$, $-CH_2-SR^8$, $-OR^8$, $-SR^8$, $-CH_2-OSO_2R^8$, $-COOR^8$ und

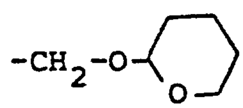 steht, wobei

$R^8$ für gegebenenfalls substituiertes Alkyl, Alkenyl, Alkinyl und Alkoxyalkyl steht und

Az für Pyrazol-1-yl, 1,2,4-Triazol-1-yl und Imidazol-1-yl steht,

Sie weisen starke fungizide Eigenschaften auf.

Man erhält die N-Oxiamide der Formel (I), wenn man

a) substituierte Aniline der Formel II

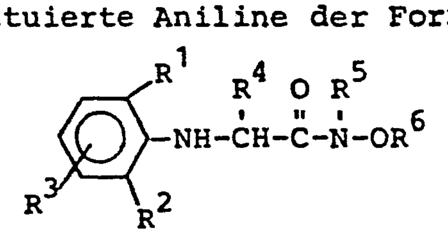

(II)

in welcher
$R^1$ bis $R^6$ die oben angegebene Bedeutung haben,

mit Säurederivaten der Formel III

$$R^7-\overset{O}{\overset{\|}{C}}-Z \qquad (III)$$

in welcher
$R^7$ die oben angegebene Bedeutung hat und
Z für Halogen oder $R^7COO$ steht,

in Gegenwart eines Verdünnungsmittels und gegebenenfalls in Gegenwart eines Säurebindemittels umsetzt, oder

b) Säuren bzw. deren Derivate der Formel IV

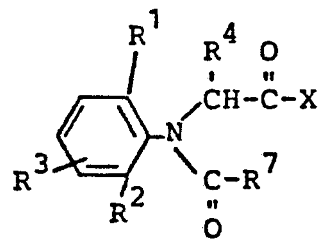

(IV)

in welcher
$R^1$-$R^4$ und $R^7$ die oben angegebene Bedeutung haben, und
X für Cl oder OH steht,
mit Hydroxylaminen bzw. deren Salzen der Formel V

$$H-\overset{R^5}{\overset{|}{N}}-OR^6 \qquad (V)$$

in welcher
$R^5$ und $R^6$ die oben angegebene Bedeutung haben

in Gegenwart eines Säurebinders und gegebenenfalls in Gegenwart eines organischen Verdünnungsmittels oder für den Fall, daß X = OH ist unter Zusatz eines

wasserabspaltenden Mittels umsetzt, oder

c) Hydroxyacetanilide der Formel VI

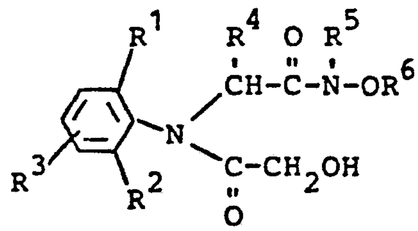

(VI)

in welcher
$R^1$ bis $R^6$ die oben angegebene Bedeutung haben,

(1) gegebenenfalls nach Aktivierung mittels Alkalimetallhydrid mit Halogeniden der Formel VII

$$Hal' - R^9 \qquad (VII)$$

in welchen
Hal' für Halogen, steht, und

$R^9$ für den Rest $R^8$ sowie die Gruppe $-SO_2R^8$ steht, wobei

$R^8$ die oben angegebene Bedeutung hat,

in Gegenwart eines Verdünnungsmittels und gegebenenfalls in Gegenwart eines Säurebinders umsetzt, oder

(2) mit Dihydropyran der Formel VIII

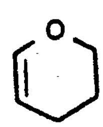

(VIII) .

in Gegenwart eines Verdünnungsmittels und gegebenenfalls in Gegenwart eines Katalysators umsetzt.

Die neuen N-Oxiamide weisen starke fungizide Eigenschaften auf. Sie zeigen dabei überraschenderweise eine erheblich höhere Wirkung als die aus dem Stand der Technik bekannten N-Chloracetyl-N-(2,6-dimethyl-phenyl)-alanin- bzw. -glycin-alkylester.

Die erfindungsgemäßen N-Oxiamide sind durch die Formel (I) allgemein definiert. In dieser Formel stehen $\underline{R^1}$, $\underline{R^2}$, $\underline{R^3}$, $\underline{R^4}$, $\underline{R^5}$ und $\underline{R^6}$ vorzugsweise für Wasserstoff und geradkettiges oder verzweigtes Alkyl mit 1 bis 4 Kohlenstoffatomen. $\underline{R^1}$ steht außerdem vorzugsweise für Halogen, wie insbesondere Fluor, Chlor oder Brom. $\underline{R^5}$ und $\underline{R^6}$ stehen außerdem vorzugsweise für gegebenenfalls durch Halogen, insbesondere Chlor substituiertes $C_{1-4}$-Alkyl, Alkenyl oder Alkinyl sowie für eine Alkylengruppierung mit 2-4 Kohlenstoffatomen. $\underline{R^7}$ steht vorzugsweise für Furyl, Tetrahydrofuryl, Thienyl, Tetrahydrothienyl, für gegebenenfalls durch Halogen, Cyano oder Thiocyano substituiertes Alkyl mit 1 bis 4 und Alkenyl sowie Alkinyl mit jeweils 2 bis 4 Kohlenstoffatomen; wobei als Halogenatome vorzugsweise Fluor und Chlor genannt seien, sowie für die Gruppierungen $-CH_2-Az$, $-CH_2-OR^8$, $-CH_2-SR^8$, $-OR^8$, $-SR^8$, $-CH_2-OSO_2R^8$, $-COOR^8$ und $-CH_2-O-$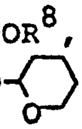.

Az steht dabei für Pyrazol-1-yl, 1,2,4-Triazol-1-yl und Imidazol-1-yl. $\underline{R^8}$ steht vorzugsweise für gegebenenfalls durch Halogen, insbesondere Fluor, Chlor und Brom, Cyano und Thiocyano substituiertes Alkyl mit 1 bis 4 und Alkenyl

sowie Alkinyl mit jeweils 2 bis 4 Kohlenstoffatomen und für Alkoxyalkyl mit 1 bis 4 Kohlenstoffatomen in jedem Alkylteil.

Ganz besonders bevorzugt sind diejenigen N-Oxiamide der Formel (I), in denen $R^1$, $R^2$ und $R^3$ für Wasserstoff, Methyl, Ethyl, Isopropyl, sek.-Butyl und tert.-Butyl stehen; $R^1$ außerdem für Chlor oder Brom steht; $R^4$ für Wasserstoff, Methyl oder Ethyl steht; $R^5$ für Wasserstoff, Methyl, Ethyl, Allyl und Propargyl und $R^6$ für Wasserstoff, Methyl, Ethyl, Allyl und Propargyl steht, wobei $R^5$ und $R^6$ miteinander auch eine $(CH_2)_n$-Brücke mit n=3 oder 4 bilden können; und $R^7$ für 2-Furyl, 2-Thienyl, 2-Tetrahydrofuryl, Methoxymethyl, Ethoxymethyl, Allyloxymethyl, Propargyloxymethyl, Methoxyethoxymethyl, Methoxymethoxymethyl, Methylmercaptomethyl, Methoxy, Ethoxy, Allyloxy, Propargyloxy, Methylsulfonyloxymethyl, Methoxycarbonyl, Ethoxycarbonyl, Allyloxycarbonyl, Propargyloxycarbonyl, Dichlormethyl, Propenyl-1, Cyclopropyl, Ethinyl, Pyrazol-1-yl-methyl, Imidazol-1-yl-methyl, 1,2,4-Triazol-1-yl-methyl und Tetrahydropyran-2-yl-oxy-methyl steht.

Im einzelnen seien außer den bei den Herstellungsbeispielen genannten Verbindungen die folgenden Verbindungen der allgemeinen Formel (I) genannt:

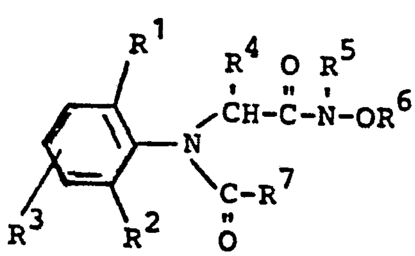

(I)

| $R^1$ | $R^2$ | $R^3$ | $R^4$ | $R^5$ | $R^6$ | $R^7$ |
|---|---|---|---|---|---|---|
| $CH_3$ | $CH_3$ | H | H | H | H | $-CH_2OCH_3$ |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |
| $CH_3$ | $CH_3$ | H | H | H | H | 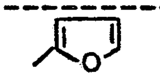 |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |
| $CH_3$ | $CH_3$ | H | H | H | H | $-CH_2-O-CH_2CH_2O-CH_3$ |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |

| $R^1$ | $R^2$ | $R^3$ | $R^4$ | $R^5$ | $R^6$ | $R^7$ |
|---|---|---|---|---|---|---|
| $CH_3$ | $CH_3$ | H | H | H | H | $-CH_2OCH_2-CH=CH_2$ |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |
| $CH_3$ | $CH_3$ | H | H | H | H | $-CH_2OCH_2-C\equiv CH$ |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |
| $CH_3$ | $CH_3$ | H | H | H | H | $-CH_2OSO_2CH_3$ |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |

| $R^1$ | $R^2$ | $R^3$ | $R^4$ | $R^5$ | $R^6$ | $R^7$ |
|---|---|---|---|---|---|---|
| $CH_3$ | $CH_3$ | H | H | H | H | $-CH=CH-CH_3$ |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |
| $CH_3$ | $CH_3$ | H | H | H | H | 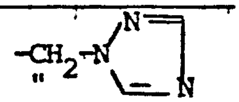 |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |
| $CH_3$ | $CH_3$ | H | H | H | H | 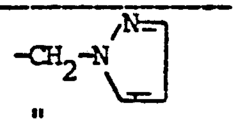 |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |

| $R^1$ | $R^2$ | $R^3$ | $R^4$ | $R^5$ | $R^6$ | $R^7$ |
|---|---|---|---|---|---|---|
| $CH_3$ | $CH_3$ | H | H | H | H | $-CH_2-$ 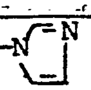 |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |
| Cl | $CH_3$ | H | H | H | H | $CH_2OCH_3$ |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |
| Cl | $CH_3$ | H | H | H | H | 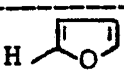 |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |

| $R^1$ | $R^2$ | $R^3$ | $R^4$ | $R^5$ | $R^6$ | $R^7$ |
|---|---|---|---|---|---|---|
| Cl | $CH_3$ | H | H | H | H | 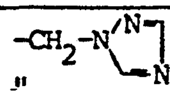 |
| " | " | H | H | H | $CH_3$ | " |
| " | " | H | H | $CH_3$ | H | " |
| " | " | H | H | $CH_3$ | $CH_3$ | " |
| " | " | H | $CH_3$ | H | H | " |
| " | " | H | $CH_3$ | H | $CH_3$ | " |
| " | " | H | $CH_3$ | $CH_3$ | H | " |
| " | " | H | $CH_3$ | $CH_3$ | $CH_3$ | " |

Verwendet man beispielsweise 2-(2', 6'-Dimethyl )-anilino-propionsäure-N,O-dimethyl-oxiamid und Methoxyacetylchlorid als Ausgangsstoffe, so kann der Reaktionsablauf durch das folgende Formelschema wiedergegeben werden (Verfahren a):

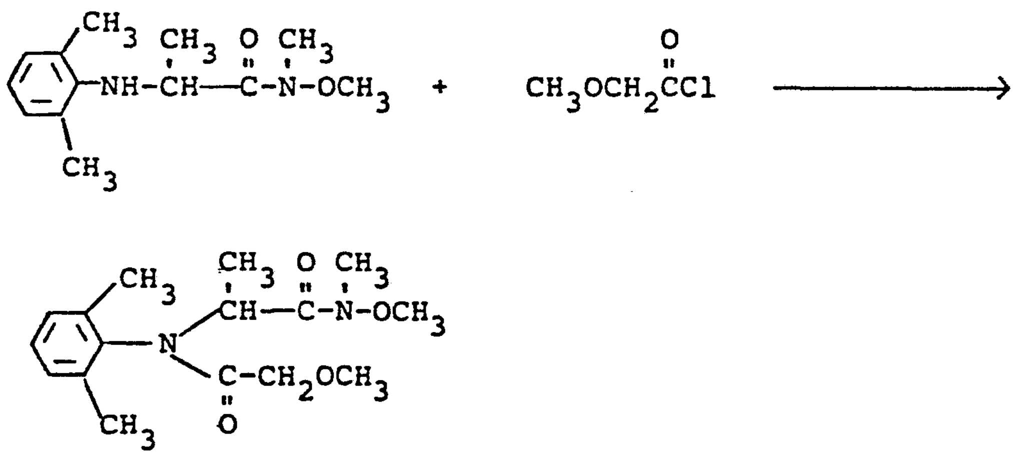

Verwendet man beispielsweise 2-(2',6'-Dimethyl)-N-methoxy-acetyl-anilino-propionsäure und O-Methylhydroxylamin in Gegenwart von Dicyclohexylcarbodiimid als Ausgangsstoffe, so kann der Reaktionsablauf durch das folgende Formelschema wiedergegeben werden (Verfahren b):

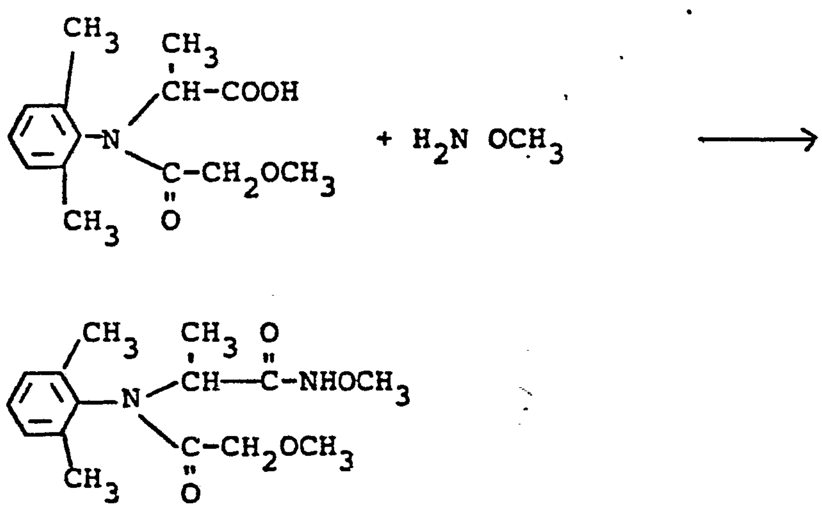

Verwendet man beispielsweise 2-(2',6'-Dimethyl)-N-hydroxyacetyl-anilino-propionsäure-N,O-dimethyl-oxiamid und Propargylbromid als Ausgangsstoffe, so kann der Reaktionsablauf durch das folgende Formelschema wiedergegeben werden (Verfahren c/1):

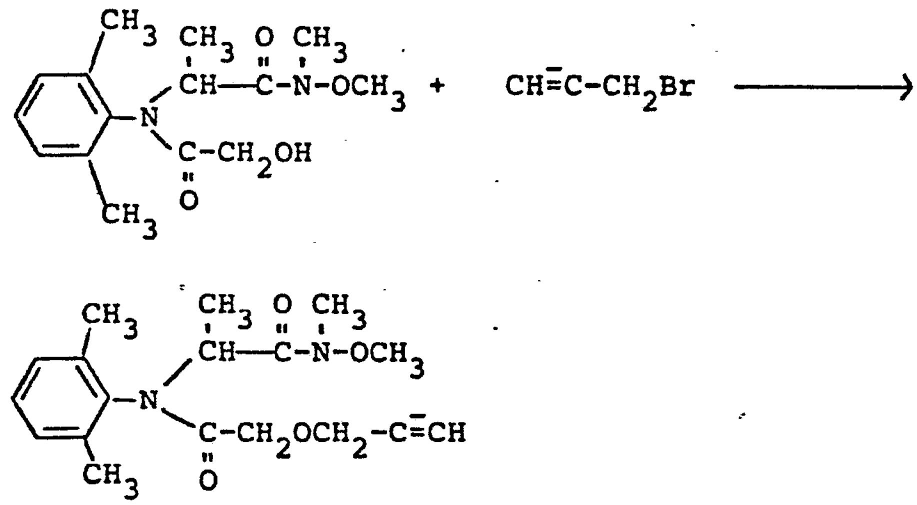

Verwendet man beispielsweise 2-(2',6'-Dimethyl)-N-hydroxyacetyl-anilino-propionsäure-N,O-dimethyl-oxiamid und 3,4-Dihydro-2H-pyran als Ausgangsstoffe, so kann der Reaktionsablauf durch das folgende Formelschema wiedergegeben werden (Verfahren c/2):

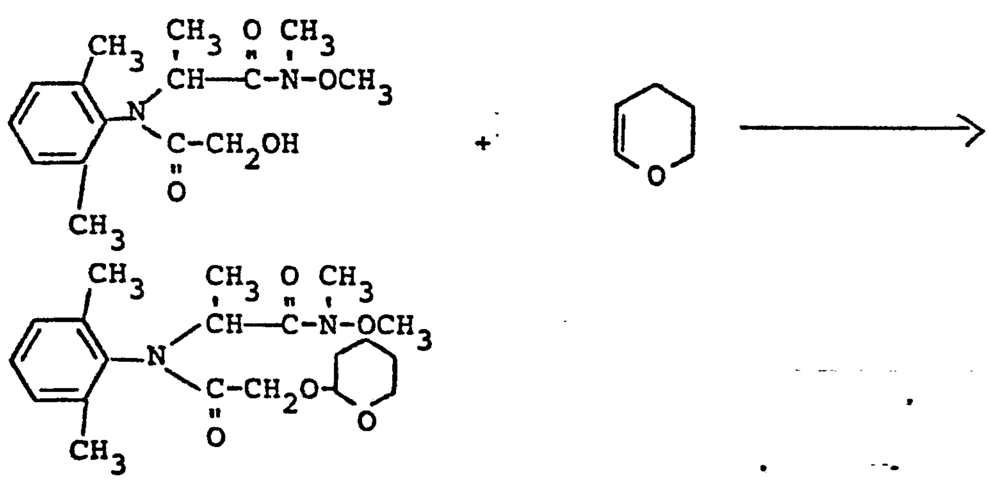

Die bei der Durchführung des erfindungsgemäßen Verfahrens (a) als Ausgangsstoffe benötigten Aniline sind durch die Formel (II) allgemein definiert. In dieser Formel stehen $R^1$, $R^2$, $R^3$, $R^4$, $R^5$ und $R^6$ vorzugsweise für diejenigen Reste, die bereits im Zusammenhang mit der Beschreibung der erfindungsgemäßen Stoffe der Formel (I) vorzugsweise für diese Reste genannt wurden. Als Beispiele für Verbindungen der Formel (II) seien im einzelnen die folgenden Verbindungen genannt:

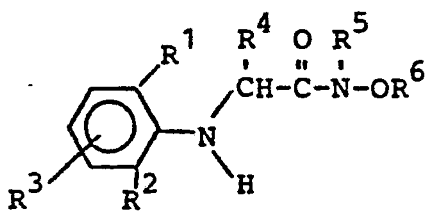

(II)

| $R^1$ | $R^2$ | $R^3$ | $R^4$ | $R^5$ | $R^6$ |
|---|---|---|---|---|---|
| $CH_3$ | $CH_3$ | H | H | H | H |
| " | " | " | H | $CH_3$ | H |
| " | " | " | " | H | $CH_3$ |
| " | " | " | " | $CH_3$ | $CH_3$ |
| " | " | " | " | H | $C_2H_5$ |
| " | " | " | " | $C_2H_5$ | H |
| " | " | " | $CH_3$ | H | H |
| " | " | " | " | $CH_3$ | H |
| " | " | " | " | H | $CH_3$ |
| " | " | " | " | $CH_3$ | $CH_3$ |
| " | " | " | " | H | $C_2H_5$ |
| " | " | " | " | $C_2H_5$ | H |
| $CH_3$ | Cl | H | H | H | H |
| " | " | H | H | $CH_2$ | H |

| $R^1$ | $R^2$ | $R^3$ | $R^4$ | $R^5$ | $R^6$ |
|---|---|---|---|---|---|
| $CH_3$ | Cl | H | H | H | $CH_3$ |
| " | " | " | " | $CH_3$ | $CH_3$ |
| " | " | " | " | H | $C_2H_5$ |
| " | " | " | " | $C_2H_5$ | H |
| " | " | " | $CH_3$ | H | H |
| " | " | " | " | $CH_3$ | H |
| " | " | " | " | H | $CH_3$ |
| " | " | " | " | $CH_3$ | $CH_3$ |
| " | " | " | " | H | $C_2H_5$ |
| " | " | " | " | $C_2H_5$ | H |
| $CH_3$ | $CH_3$ | " | $CH_3$ | $-(CH_2)_3-$ | |
| $CH_3$ | Cl | " | $CH_3$ | " | |
| $CH_3$ | $CH_3$ | " | " | $-(CH_2)_4-$ | |
| $CH_3$ | Cl | " | " | $-(CH_2)_4-$ | |

Die substituierten Aniline der Formel (II) sind noch nicht bekannt. Sie können erhalten werden, indem man z.B. Aniline der Formel IX

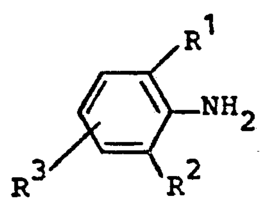

(IX)

in welcher

$R^1$, $R^2$ und $R^3$ die oben angegebene Bedeutung haben, mit substituierten Oxiamiden der Formel (X)

$$\begin{array}{l} \quad\;\; R^4 \;\; O \;\; R^5 \\ \quad\;\;\; | \quad\; \| \quad\; | \\ Y-CH-C-N-OR^6 \end{array} \qquad (X)$$

in welcher

$R^4$, $R^5$ und $R^6$ die oben angegebene Bedeutung haben, und

Y für Chlor oder Brom steht,

in Gegenwart eines Säurebinders, wie beispielsweise Alkalicarbonate und gegebenenfalls in Gegenwart eines inerten organischen Lösungsmittels, wie beispielsweise Toluol oder Dimethylformamid, bei Temperaturen zwischen 20 und 160°C umsetzt.

Die als Ausgangsstoffe benötigten Aniline der Formel (IX) sind allgemein bekannte Verbindungen der organischen Chemie. Als Beispiele seien genannt:

Anilin; 2,3-Dimethylanilin; 2,5-Dimethylanilin; 3,5-Dimethylanilin; 2,6-Diethylanilin; 2-Ethyl-6-methylanilin; 2,3,4-Trimethylanilin; 2,4,6-Trimethylanilin; 2,4,5-Trimethylanilin; 2-Ethyl-4,6-dimethylanilin; 2,6-Diethyl-4-methylanilin; 2,6-Diisopropyl-4-methylanilin; 2,3,5-Trimethylanilin; 2,3,6-Trimethylanilin; 6-Chlor-2-methyl-anilin; 2-Brom-6-methylanilin; 2-Chlor-6-tert.-butyl-anilin.

Die außerdem als Ausgangsstoffe benötigten substituierten Oxiamide der Formel (X) sind teilweise bekannt (vgl. US-Patentschrift 3 835 205, 3 917 664) bzw. lassen sich

durch Umsetzung der α-Halogencarbonsäurechloride der Formel (XI)

$$R^4-\underset{\displaystyle Y}{\underset{|}{CH}}-\overset{\displaystyle O}{\overset{\|}{C}}-Cl \qquad (XI)$$

in welcher

$R^4$ und Y die oben angegebene Bedeutung haben

mit den Hydroxylaminen der Formel (V)

$$H-\overset{\displaystyle R^5}{\overset{|}{N}}-OR^6 \qquad (V)$$

in welcher

$R^5$ und $R^6$ die oben angegebene Bedeutung haben,

leicht herstellen.

Die außerdem für das erfindungsgemäße Verfahren (a) als Ausgangsstoffe zu verwendenden Säurederivate d.h. -chloride oder -bromide, bzw. Anhydride sind durch die Formel (III) allgemein definiert. In dieser Formel steht $R^7$ vorzugsweise für diejenigen Reste, die bereits im Zusammenhang mit der Beschreibung der erfindungsgemäßen Stoffe der Formel (I) vorzugsweise für diesen Substituenten genannt wurden.

Die Säurechloride oder -bromide bzw. -anhydride der

Formel (III) sind allgemein bekannte Verbindungen der organischen Chemie.

Die bei der Durchführung des erfindungsgemäßen Verfahrens (b) als Ausgangsstoffe benötigten Säuren bzw. deren Derivate sind durch die Formel IV allgemein definiert. In dieser Formel stehen $\underline{R^1}$, $\underline{R^2}$, $\underline{R^3}$ und $\underline{R^7}$ vorzugsweise für diejenigen Reste, die bereits im Zusammenhang mit der Beschreibung der erfindungsgemäßen Stoffe der Formel (I) vorzugsweise für diese Substituenten genannt wurden.

Die Carbonsäuren bzw. deren Derivate der Formel IV können erhalten werden, indem man die entsprechenden, aus DE-OS 2 515 091 bekannten, Methyl- bzw. Ethylester verseift und die dabei erhaltenen Säuren gegebenenfalls mit Thionylchlorid oder Oxalylchlorid gegebenenfalls in einem Verdünnungsmittel umsetzt.

Als Beispiele für die Ausgangsstoffe der Formel IV seien genannt:

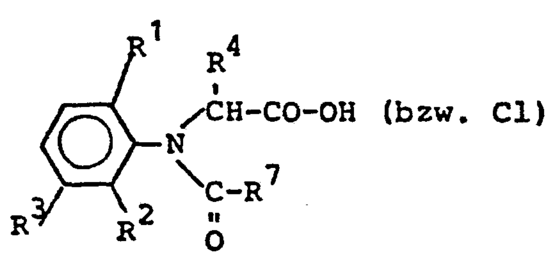

(IV)

| $R^1$ | $R^2$ | $R^3$ | $R^4$ | $R^7$ |
|---|---|---|---|---|
| $CH_3$ | $CH_3$ | H | H | $CH_2O-CH_3$ |
| " | " | " | H | 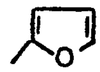 |
| " | " | " | " | $CH_2OCH_2CH_2OCH_3$ |
| " | " | " | " | $CH_2OCH_2-CH=CH_2$ |
| " | " | " | " | $CH_2OCH_2C\equiv CH$ |
| " | " | " | " | $CH_2OSO_2CH_3$ |
| " | " | " | " | $CH_2-$ 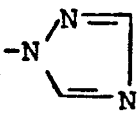 |
| " | " | " | $CH_3$ | $CH_2OCH_3$ |
| " | " | " | " | 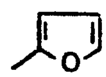 |
| " | " | " | " | $-CH_2OCH_2CH_2OCH_3$ |
| " | " | " | " | $-CH_2OCH_2CH=CH_2$ |
| " | " | " | $CH_3$ | $-CH_2OCH_2-C\equiv CH$ |
| " | " | " | " | $-CH_2OSO_2CH_3$ |
| " | " | " | " | $CH_2-$ 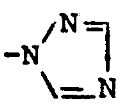 |
| " | " | " | " | $-CH=CH-CH_3$ |
| $CH_3$ | Cl | H | H | $CH_2OCH_3$ |
| " | " | " | " | 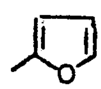 |
| " | " | " | " | $CH_2OCH_2CH_2OCH_3$ |
| " | " | " | " | $-CH_2OCH_2-CH=CH_2$ |
| " | " | " | " | $-CH_2OCH_2-C\equiv CH$ |
| " | " | " | " | $-CH_2-OSO_2CH_3$ |

| $R^1$ | $R^2$ | $R^3$ | $R^4$ | $R^7$ |
|---|---|---|---|---|
| " | " | " | " | 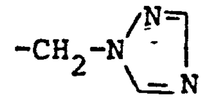 |
| " | " | " | $CH_3$ | $-CH_2OCH_3$ |
| " | " | " | $CH_3$ | 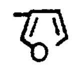 |
| " | " | " | $CH_3$ | $-CH_2OCH_2CH_2OCH_3$ |
| " | " | " | $CH_3$ | $-CH_2OCH_2CH{=}CH_2$ |
| " | " | " | " | $-CH_2OCH_2-C{\equiv}CH$ |
| " | " | " | " | $-CH_2OSO_2CH_3$ |
| " | " | " | " | 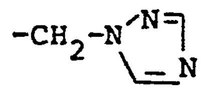 |

Die bei der Durchführung des erfindungsgemäßen Verfahrens (c1) als Ausgangsstoffe benötigten Hydroxyacetanilide sind durch die Formel (VI) allgemein definiert. In dieser Formel stehen $\underline{R}^1$, $\underline{R}^2$, $\underline{R}^3$, $\underline{R}^4$, $\underline{R}^5$ und $\underline{R}^6$ vorzugsweise für diejenigen Reste, die bereits im Zusammenhang mit der Beschreibung der erfindungsgemäßen Stoffe der Formel (I) vorzugsweise für diese Substituenten genannt werden.

Die Hydroxyacetanilide der Formel (VI) können auf allgemein bekannte Art und Weise erhalten werden, indem man Acyloxyacetylanilide der Formel

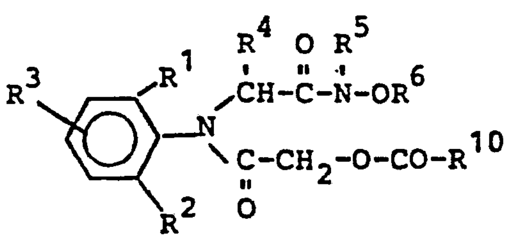

(XIII)

in welcher

$R^1$, $R^2$, $R^3$, $R^4$, $R^5$ und $R^6$ die oben angegebene Bedeutung haben und

$R^{10}$ für Alkyl mit 1 bis 4 Kohlenstoffatomen steht,

mit Natron- oder Kalilauge bzw. mit einem Alkalialkoholat eines niederen Alkohols wie z.B. Natriummethylat oder Natriumethylat, bei Temperaturen zwischen 20 und 40°C verseift und nach Neutralisation in üblicher Weise die Verbindungen der Formel (VI) isoliert.

Die Acyloxyacetylanilide der Formel (XIII) können in allgemein üblicher und bekannter Art und Weise erhalten werden, indem man Aniline der Formel (II) mit entsprechenden Acyloxyacetylhalogeniden umsetzt.

Die außerdem für das erfindungsgemäße Verfahren (c/1) als Ausgangsstoffe zu verwendenden Halogenide sind durch die Formel (VII) allgemein definiert. In dieser Formel steht $R^9$ bzw. $R^8$ vorzugsweise für die Reste, die bereits im Zusammenhang mit der Beschreibung der erfindungsgemäßen Stoffe der Formel (I) vorzugsweise für diesen Substituenten genannt wurden. Hal steht vorzugsweise für Chlor, Brom oder Jod.

Die Halogenide der Formel (VII) sind allgemein bekannte Verbindungen der organischen Chemie.

Das für das erfindungsgemäße Verfahren (c/2) außerdem als Ausgangsstoff zu verwendende Dihydropyran ist ebenfalls eine bekannte Verbindung der organischen Chemie.

Als Verdünnungsmittel kommen für die erfindungsgemäße Umsetzung gemäß Verfahren (a) organische Lösungsmittel in Frage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; aliphatische und aromatische Kohlenwasserstoffe, wie Petrolether, Benzol, Toluol oder Xylol; halogenierte Kohlenwasserstoffe, wie Methylenchlorid, Tetrachlorkohlenstoff, Chloroform oder Chlorbenzol; und Ester, wie Essigester.

Das erfindungsgemäße Verfahren (a) kann gegebenenfalls in Gegenwart von Säurebindern (Halogenwasserstoff-Akzeptoren) durchgeführt werden. Als solche können alle üblichen Säurebindemittel verwendet werden. Hierzu gehören vorzugsweise organische Basen, wie tertiäre Amine, beispielsweise Triethylamin, oder Pyridin, ferner anorganische Basen, wie beispielsweise Alkalihydroxide und Alkalicarbonate.

Die Reaktionstemperaturen können bei der Durchführung des erfindungsgemäßen Verfahrens (a) in einem größeren Bereich variiert werden. Im allgemeinen arbeitet man zwischen 0°C und 120°C, vorzugsweise zwischen 20 und 100°C.

Bei der Durchführung des erfindungsgemäßen Verfahrens (a) setzt man vorzugsweise auf 1 Mol der Verbindungen der Formel (II) 1 bis 1,5 Mol Acylierungsmittel und 1 bis 1,5 Mol Säurebinder ein. Die Isolierung der Verbindungen der Formel (I) erfolgt in üblicher Weise.

Als Verdünnungsmittel kommen für die erfindungsgemäße Umsetzung gemäß Verfahren (b) organische Lösungsmittel in Frage. Hierzu gehören vorzugsweise die bei dem Verfahren (a) bereits genannten Solventien.

Die Reaktionstemperaturen können bei der Durchführung des erfindungsgemäßen Verfahrens (b) in einem größeren Bereich variiert werden. Im allgemeinen arbeitet man zwischen 0°C und 80°C, vorzugsweise zwischen 0°C und 50°C. Als wasserabspaltendes Mittel wird z.B. Dicyclohexylcarbodiimid eingesetzt.

Bei der Durchführung des erfindungsgemäßen Verfahrens (b) arbeitet man vorzugsweise mit äquimolaren Mengen. Es ist jedoch auch möglich, die Hydroxylamine der Formel (V) im Überschuß einzusetzen. Die Aufarbeitung und Isolierung der Reaktionsprodukte erfolgt nach üblichen Methoden.

Die Kupplung der Säure (IV) mit den Hydroxylaminen (V) unter Mitwirkung des wasserabspaltenden Reagens Dicyclohexylcarbodiimid erfolgt nach der üblichen Methode (vgl. Chemiker-Zeitung 103 (1979) S. 19). Bevorzugt werden äquimolare Mengen der Reaktionspartner eingesetzt. Als Verdünnungsmittel kommen inerte organische Lösungsmittel wie z.B. Methylenchlorid, Dioxan oder Toluol in Frage. Die Reaktionstemperaturen können in einem größeren Bereich variiert werden. Im allgemeinen arbeitet man zwischen 0°C und 50°C, vorzugsweise zwischen 20°C und 30°C.

Als Verdünnungsmittel kommen für die erfindungsgemäße Umsetzung gemäß Verfahren (c) organische Lösungsmittel in Frage. Hierzu gehören vorzugsweise die beim Verfahren (a) bereits genannten Lösungsmittel.

Die Umsetzung nach Verfahren (c/1) kann gegebenenfalls in Gegenwart eines Säurebinders durchgeführt werden. Man kann alle üblicherweise verwendbaren anorganischen oder organischen Säurebinder zugeben. Hierzu gehören vorzugsweise die beim Verfahren (a) bereits genannten Verbindungen.

Die Reaktionstemperaturen können beim Verfahren (c/1) in einem größeren Bereich variiert werden. Im allgemeinen arbeitet man zwischen 20 und 150°C, vorzugsweise bei der Siedetemperatur des Lösungsmittels, beispielsweise zwischen 60 und 100°C.

Bei der Durchführung des erfindungsgemäßen Verfahrens (c/1) setzt man auf 1 Mol der Verbindungen der Formel (VI), gegebenenfalls nach Zugabe von 1 bis 2 Mol einer starken Base, wie z.B. eines Alkalihydrids, 1 Mol Halogenid der Formel (VII) und gegebenenfalls 1 bis 2 Mol Säurebinder ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt und in üblicher Weise aufgearbeitet.

Nach einer bevorzugten Ausführungsform des Verfahrens

(c/1) wird zweckmäßigerweise so verfahren, daß man von einem Hydroxyacetanilid der Formel (VI) ausgeht, letzteres in einem geeigneten inerten Lösungsmittel mittels Alkalimetall-hydrid in das Alkalimetallalkanolat überführt, und letzteres ohne Isolierung sofort mit einem Halogenid der Formel (VII) umsetzt, wobei unter Austritt von Alkalihalogenid die erfindungsgemäßen Verbindungen der Formel (I) in einem Arbeitsgang erhalten werden.

Die Umsetzung nach Verfahren (c/2) kann gegebenenfalls in Gegenwart eines Katalysators durchgeführt werden. Hierzu verwendet man vorzugsweise Chlorwasserstoff (vgl. J. Am. Chem. Soc. 69, 2246 (1947), ibid. 70, 4187 (1948)).

Die Reaktionstemperaturen können beim Verfahren (c/2) in einem größeren Bereich variiert werden. Im allgemeinen arbeitet man zwischen 0 und 100°C, vorzugsweise zwischen 20 und 60°C.

Bei der Durchführung des erfindungsgemäßen Verfahrens (c/2) arbeitet man vorzugsweise in molaren Mengen. Die Isolierung der Verbindungen der Formel (I) erfolgt in üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo -Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

1.

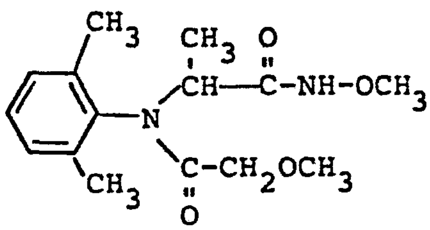

Eine Mischung aus 26,5 g (0,1 Mol) N-(2,6-dimethyl-phenyl)-N-methoxy-acetyl-alanin, 9,6 g (0,11 Mol) O-Methylhydroxylamin-hydrochlorid und 11 g (0,11 Mol) Triethylamin wird unter Rühren in 150 ml Methylenchlorid tropfenweise mit einer Lösung von 20,6 g (0,1 Mol) Dicyclohexylcarbodiimid in 100 ml Methylenchlorid versetzt. Anschließend wird noch 5 Stunden bei 20°C gerührt, der auskristallisierte Harnstoff abfiltriert und das Filtrat nacheinander mit Wasser, gesättigter Natriumhydrogencarbonat-Lösung und wieder Wasser gewaschen. Nach Trocknen über Natriumsulfat wird das Lösungsmittel im Vakuum abgezogen und der Rückstand mit Ether kristallisiert.

Ausbeute: 10,5 g (36 % d.Th.)

Fp.: 132-133°C.

2.

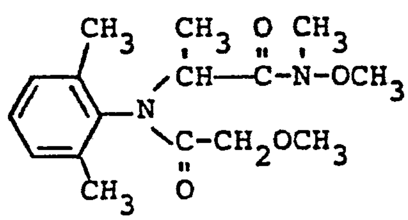

5,5 g (0,018 Mol) der Verbindung aus Beispiel 1 und 100 mg

Triethylbenzylammoniumchlorid als Katalysator werden in einem Zweiphasengemisch aus 20 ml 50 %iger Natronlauge und 100 ml Toluol unter schnellem Rühren tropfenweise mit 3,2 g (0,023 Mol) Dimethylsulfat versetzt. Nach 5-stündigem Rühren bei Raumtemperatur wird die wäßrige Phase abgetrennt, die organische Phase mehrmals mit Wasser gewaschen und das Lösungsmittel im Vakuum abgezogen. Der Rückstand wird mit Diisopropylether kristallisiert.
Ausbeute: 2,5 g (45 % d. Th.)
Fp.: 55 - 56°C.

3. 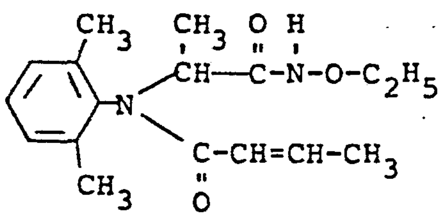

Zu einer Lösung von 4,6 g (0,02 Mol) N-(2,6-dimethylphenyl)-alanin-N'-ethoxy-amid in 50 ml Dichlormethan und 4,7 g (0,06 Mol) Pyridin werden bei Raumtemperatur 4,5 g (0,04 Mol, Überschuß) Crotonsäurechlorid getropft. Nach Stehenlassen über Nacht und zweimaligem Waschen mit je 30 ml Wasser wird mit Natriumsulfat getrocknet, das Lösungsmittel im Vakuum abgezogen, der Rückstand auf 20 g Kieselgel aufgezogen und mit Cyclohexan/Aceton in ansteigender Polarität wieder eluiert. Die Hauptfraktion liefert ein helles Öl, das nach Verrühren mit Diisopropylether kristallisiert.
Ausbeute 2 g (34 %)
Fp. 152-155°C

Entsprechend den Beispielen 1 bis 3 werden die folgenden Verbindungen der allgemeinen Formel

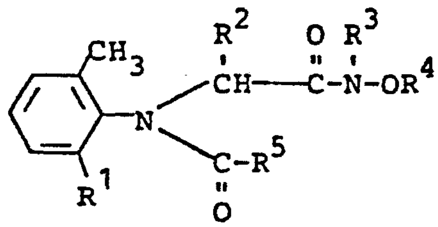

erhalten.

| Bsp. Nr. | $R^1$ | $R^2$ | $R^3$ | $R^4$ | $R^5$ | Fp. |
|---|---|---|---|---|---|---|
| 4 | $CH_3$ | $CH_3$ | H | $C_2H_5$ | 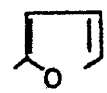 | 156-57°C |
| 5 | $CH_3$ | $CH_3$ | $CH_3$ | $C_2H_5$ | 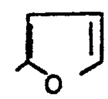 | 89-90°C |
| 6 | $CH_3$ | $CH_3$ | $CH_3$ | $CH_3$ | 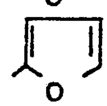 | 138-40°C |
| 7 | $CH_3$ | $CH_3$ | H | $CH_3$ | 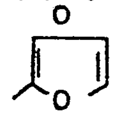 | 170-71°C |

| Nr. | $R^1$ | $R^2$ | $R^3$ | $R^4$ | $R^5$ | Physik. Konst. |
|---|---|---|---|---|---|---|
| 8 | Cl | $CH_3$ | H | $CH_3$ | $CH_2OCH_3$ | $n_D^{20}$ = 1.5159 |
| 9 | Cl | $CH_3$ | H | $C_2H_5$ | $CH_2OCH_3$ | Fp. = 98-104°C |
| 10 | Cl | $CH_3$ | H | $C_2H_5$ | $CH{=}CH{-}CH_3$ | Fp. = 143-145°C |
| 11 | Cl | $CH_3$ | H | $C_2H_5$ | 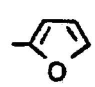 | Fp. = 165-168°C |

| Bsp. Nr. | $R^1$ | $R^2$ | $R^3$ | $R^4$ | $R^5$ | Physik. Konst. |
|---|---|---|---|---|---|---|
| 12 | $CH_3$ | H | H | H | $CH_2OCH_3$ | Fp. = 120°C (Zers.) |
| 13 | $C_2H_5$ | H | H | H | $CH_2OCH_3$ | $n_D^{20}$ = 1.5348 |
| 14 | Cl | H | H | H | $CH_2OCH_3$ | $n_D^{20}$ = 1.5381 |
| 15 | Cl | $CH_3$ | H | H | $CH_2OCH_3$ | $n_D^{20}$ = 1.5227 |
| 16 | $CH_3$ | $CH_3$ | H | H | $CH_2OCH_3$ | Fp. = 62°C |
| 17 | $CH_3$ | H | H | H | $CH=CH-CH_3$ | Fp. = 65-72°C |

Beispiel

Phytophthora-Test (Tomate) / protektiv / systemisch

Lösungsmittel: 4,7 Gewichtsteile: Aceton
Emulgator: 0,3 Gewichtsteile: Alkylarylpolyglycolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Zur Prüfung auf systemische Eigenschaften wird die Wirkstoffzubereitung auf Einheitserde gegossen, in der sich junge versuchsbereite Pflanzen befinden. 3 Tage nach der Behandlung werden die Pflanzen mit einer wäßrigen Sporensuspension von Phytophthora infestans inokuliert.

Die Pflanzen werden in einer Inkubationskabine mit 100 % relativer Luftfeuchtigkeit bei ca. 20°C aufgestellt.

4 Tage nach der Inkulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 1,2,6.

Patentansprüche

1. N-Oxiamide der allgemeinen Formel I

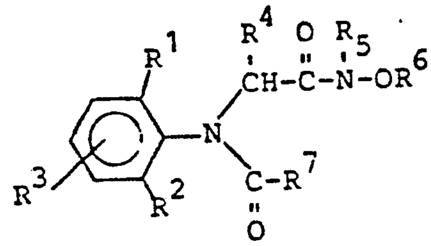

(I)

in welcher

$R^1$ für Wasserstoff, Alkyl oder Halogen steht,

$R^2$ für Wasserstoff oder Alkyl steht,

$R^3$ für Wasserstoff oder Alkyl steht,

$R^4$ für Wasserstoff oder Alkyl steht,

$R^5$ für Wasserstoff, gegebenenfalls durch Halogen substituiertes Alkyl, Alkenyl, Alkinyl steht,

$R^6$ für Wasserstoff, gegebenenfalls durch Halogen substituiertes Alkyl, Alkenyl, Alkinyl steht, wobei $R^5$ und $R^6$ auch eine Alkylenbrücke bilden können,

$R^7$ für Furyl, Tetrahydrofuryl, Thienyl, Tetrahydrothienyl, gegebenenfalls durch Halogen, Cyano oder Thiocyano substituiertes Alkyl, Alkenyl und

Alkinyl; sowie die Gruppierungen $-CH_2-Az$, $-CH_2-OR^8$, $-CH_2-SR^8$, $-OR^8$, $-SR^8$, $-CH_2-OSO_2R^8$, $-COOR^8$ und $-CH_2-O-$ 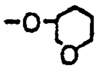 steht, wobei

$R^8$ für gegebenenfalls substituiertes Alkyl, Alkenyl, Alkinyl und Alkoxyalkyl steht, und

Az für Pyrazol-1-yl, 1,2,4-Triazol-1-yl und Imidazol-1-yl steht.

2. Verfahren zur Herstellung der N-Oxiamide der Formel (I)

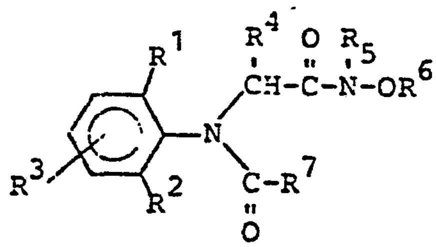

(I),

in welcher

$R^1$ für Wasserstoff, Alkyl oder Halogen steht,

$R^2$ für Wasserstoff oder Alkyl steht,

$R^3$ für Wasserstoff oder Alkyl steht,

$R^4$ für Wasserstoff oder Alkyl steht,

$R^5$ für Wasserstoff, gegebenenfalls durch Halogen substituiertes Alkyl, Alkenyl, Alkinyl steht,

$R^6$ für Wasserstoff, gegebenenfalls durch Halogen substituierte Alkyl, Alkenyl, Alkinyl steht, wobei $R^5$ und $R^6$ auch eine Alkylenbrücke bilden können,

$R^7$ für Furyl, Tetrahydrofuryl, Thienyl, Tetrahydrothienyl, gegebenenfalls durch Halogen, Cyano oder Thiocyano substituiertes Alkyl, Alkenyl und Alkinyl; sowie die Gruppierungen $-CH_2-Az$, $-CH_2-OR^8$, $-CH_2-SR^8$, $-OR^8$, $-SR^8$, $-CH_2-OSO_2R^8$, $-COOR^8$ und $-CH_2-O-$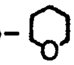 steht

$R^8$ für gegebenenfalls substituiertes Alkyl, Alkenyl, Alkinyl und Alkoxyalkyl steht und

Az für Pyrazol-1-yl, 1,2,4-Triazol-1-yl und Imidazol-1-yl steht,

dadurch gekennzeichnet, daß man

a) substituierte Aniline der Formel II

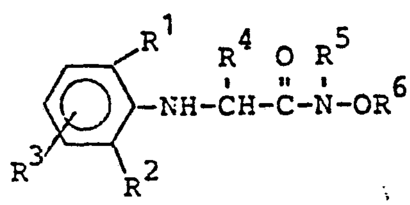

(II)

in welcher

$R^1$ bis $R^6$ die oben angegebene Bedeutung haben,

mit Säurederivaten der Formel III

$$R^7-\overset{O}{\overset{\|}{C}}-Z \qquad (III)$$

in welcher

$R^7$ die oben angegebene Bedeutung hat und

Z für Halogen oder $R^7COO$ steht,

in Gegenwart eines Verdünnungsmittels und gegebenenfalls in Gegenwart eines Säurebindemittels umsetzt, oder

b) Säuren bzw. deren Derivate der Formel IV

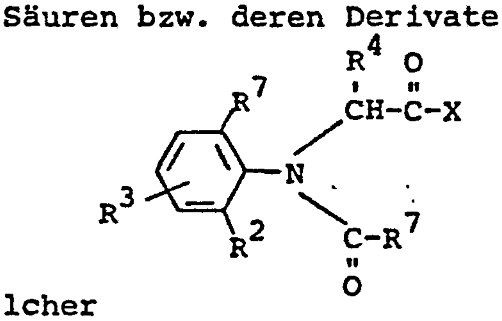

(IV)

in welcher

$R^1$ bis $R^4$ und $R^7$ die oben angegebene Bedeutung haben, und

X für Cl oder OH steht,

mit Hydroxylaminen bzw. deren Salze der Formel V

$$H-\overset{R^5}{\overset{|}{N}}-OR^6 \qquad (V)$$

in welcher

$R^5$ und $R^6$ die oben angegebene Bedeutung haben

in Gegenwart eines Säurebinders und gegebenenfalls in Gegenwart eines organischen Verdünnungsmittels oder

für den Fall, daß X = OH ist, unter Zusatz eines wasserabspaltenden Mittels umsetzt oder

c) Hydroxyacetanilide der Formel VI

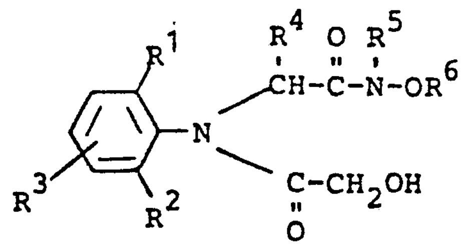

(VI)

in welcher

$R^1$ bis $R^6$ die oben angegebene Bedeutung haben,

(1) gegebenenfalls nach Aktivierung mittels Alkalimetallhydrid mit Halogeniden der Formel VII

$$Hal' - R^9 \qquad (VII)$$

in welcher

Hal' für Halogen steht und

$R^9$ für den Rest $R^8$ sowie die Gruppe $-SO_2R^8$ steht, wobei

$R^8$ die oben angegebene Bedeutung hat,

in Gegenwart eines Verdünnungsmittels und gegebenenfall in Gegenwart eines Säurebinders umsetzt, oder

(2) mit Dihydropyran der Formel VIII

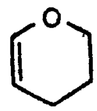

(VIII)

in Gegenwart eines Verdünnungsmittels und gegebenenfalls in Gegenwart eines Katalysators umsetzt.

3. N-Oxiamide der Formel I, in der $\underline{R}^1$, $\underline{R}^2$, $\underline{R}^3$, $\underline{R}^4$, $\underline{R}^5$ und $\underline{R}^6$ unabhängig voneinander für Wasserstoff und geradkettiges oder verzweigtes Alkyl mit 1 bis 4 Kohlenstoffatomen stehen, $\underline{R}^1$ außerdem für Halogen, $\underline{R}^5$ und $\underline{R}^6$ außerdem für gegebenenfalls durch Halogen substituiertes Alkyl, Alkenyl oder Alkinyl sowie für eine Alkylengruppierung mit 2 - 4 Kohlenstoffatomen stehen, $\underline{R}^7$ für Furyl, Tetrahydrofuryl, Thienyl, Tetrahydrothienyl, für gegebenenfalls durch Halogen, Cyano oder Thiocyano substituiertes Alkyl mit 1 bis 4 C-Atomen und Alkenyl sowie Alkinyl mit jeweils 2 bis 4 Kohlenstoffatomen, sowie für die Gruppierungen $-CH_2-Az$, $-CH_2-OR^8$, $-CH_2-SR^8$, $-OR^8$, $-SR^8$, $-CH_2-OSO_2R^8$, $-COOR^8$ und $-CH_2-O-$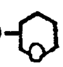 steht, wobei $\underline{Az}$ für Pyrazol-1-yl, 1,2,4-Triazol-1-yl und Imidazol-1-yl, $\underline{R}^8$ für gegebenenfalls durch Haolgen, Cyano und Thiocyano substituiertes Alkyl mit 1 bis 4 C-Atomen und Alkenyl sowie Alkinyl mit jeweils 2 bis 4 Kohlenstoffatomen und für Alkoxyalkyl mit 1 bis 4 Kohlenstoffatomen in jedem Alkylteil stehen.

4. N-Oxiamide der Formel I, in der $R^1$, $R^2$ und $R^3$ unabhängig voneinander für Wasserstoff, Methyl, Ethyl, Isopropyl, sek.-Butyl und tert.-Butyl stehen; R' außerdem für Chlor oder Brom steht; $R^4$ für Wasserstoff, Methyl oder Ethyl steht; $R^5$ für Wasserstoff und $R^6$ für Wasserstoff, Methyl, Ethyl, Allyl und Propargyl stehen, wobei $R^5$ und $R^6$ miteinander auch eine $(CH_2)_n$-Brücke mit n = 3 oder 4 bilden können; und $R^7$ für 2-Furyl, 2-Thienyl, 2-Tetrahydrofuryl, Methoxymethyl, Ethoxymethyl, Allyloxymethyl, Propargyloxymethyl, Methoxyethoxymethyl, Methoxymethoxymethyl, Methylmercaptomethyl, Methoxy, Ethoxy, Allyloxy, Propargyloxy, Methylsulfonyloxymethyl, Methoxycarbonyl, Ethoxycarbonyl, Allyloxycarbonyl, Propargyloxycarbonyl, Dichlormethyl, Propenyl-1, Cyclopropyl, Ethinyl, Pyrazol-1-yl-methyl, Imidazol-1-yl-methyl, 1,2,4-Triazol-1-yl-methyl und Tetrahydropyran-2-yl-oxy-methyl steht.

5. Fungizide Mittel, gekennzeichnet durch einen Gehalt an mindestens einem N-Oxiamid der Formel (I).

6. Verwendung von N-Oxiamide der Formel (I) zur Bekämpfung von pflanzenperthogenen Pilzen.

7. Verfahren zur Bekämpfung von pflanzenperthogenen Pilzen, dadurch gekennzeichnet, daß man N-Oxiamide der Formel (I) auf pflanzenperthogenen Pilze oder ihren Lebensraum einwirken läßt.

8. Verfahren zur Herstellung fungizider Mittel, dadurch gekennzeichnet, daß man N-Oxiamide der Formel (I) mit Streckmitteln und/oder oberflächenaktiven Mitteln vermischt.